# EUROPEAN PATENT APPLICATION

(11) **EP 1 475 392 A1**
(43) Date of publication of application: **10.11.2004**
(21) Application number: 03737444.4
(22) Date of filing: 30.01.2003
(51) Int. Cl.: C08B 37/08, A23L 1/312, A23L 1/30

(54) **MUCOPOLYSACCHARIDES AND PROCESS FOR PRODUCING THE SAME**

(30) Priority: 05.02.2002 JP 2002027932; 01.10.2002 JP 2002288947
(71) Applicant: Nippon Barrier Free Co. Ltd., Tokyo 101-0051 (JP)
(72) Inventor: KACHI, Gasho, Ota-ku, Tokyo 146-0094 (JP)
(74) Representative: Turi, Michael, Dipl.-Phys.
(86) International application number: PCT/JP2003/000890
(87) International publication number: WO 2003/066683

(57) **Abstract**

Cartilage of fish is treated with enzyme or alkali so as to obtain an aqueous solution, which is then defatted, deodorized, decolorized, filtrated, and dried, such that a mucopolysaccharide is produced. Alternatively, cartilage of fish is pressure-treated so as to obtain an aqueous solution, which is then treated with enzyme, defatted, deodorized, decolorized, filtrated, and dried, such that a mucopolysaccharide is produced. According to the present invention, a mucopolysaccharide such as chondroitin sulfuric acid can be produced in a short time and at a low cost. Further, the mucopolysaccharide produced is used as a cosmetic material or food material, for example.

## Description

### Technical Field

The present invention relates to a mucopolysaccharide produced from cartilage of fish.

### Background Art

It is verified by the past researches that the cartilage of fish such as nose cartilage of salmon, for example, includes the mucopolysaccharide such as chondroitin sulfuric acid. And recently, it is considered that the chondroitin sulfuric acid derived from nose cartilage of salmon is used for cosmetics and foods.

Conventionally, as methods to produce a mucopolysaccharide from nose cartilage of salmon, there are Japanese Patent Application Laid-open No. 2001-231497 and Japanese Patent Application Laid-open No. 2001-247602. In these prior arts, the nose cartilage of salmon or the like is defatted by being mashed at a low temperature of minus 30°C to minus 60°C, and thereafter treated with alkali, heated, treated with enzyme. Then the digestive juice thereof is ethanol-precipitated, filtrated, centrifuged, and dried, and further dissolved by the ion-exchange resin, filtrated, and thereafter freeze-dried, so that the mucopolysaccharide such as the chondroitin sulfuric acid is obtained.

However, in the conventional production methods, the use of the ion-exchange resin for the filtration requires a large amount of ethanol, so that the production cost becomes higher, and the mucopolysaccharide becomes considerably expensive. In addition, the ethanol precipitation, filtration, and centrifugation are repeated several times so that the production time gets longer.

### Disclosure of the Invention

Accordingly, it is an object of the present invention to provide a method to allow production of a mucopolysaccharide at a cost as low, and in a time as short, as possible.

In order to attain the object, according to the present invention, is provided a method to produce a mucopolysaccharide, the method characterized in defatting, deodorizing, decolorizing, filtrating, and drying an aqueous solution obtained by treating cartilage of fish with enzyme or alkali. Further, according to the present invention, is provided a method to produce a mucopolysaccharide, the method characterized in treating with enzyme an aqueous solution obtained by pressure-treating cartilage of fish, and defatting, deodorizing, decolorizing, filtrating, and drying the aqueous solution.

Further, in the present invention, a mucopolysaccharide is characterized in that it is produced by the aforesaid production method. The mucopolysaccharide thus produced is used as a cosmetic material or a food material, as an example.

In the present invention, presented as examples of the fish are shark, ray, salmon, and so forth. Also presented as an example of cartilage of fish is the nose cartilage of salmon. In the present invention, the mucopolysaccharide is produced, for example, by treating with enzyme an aqueous solution obtained by dissolving cartilage of fish pressured at about 120°C for one hour, which is then defatted, deodorized, decolorized, filtrated and dried. According to the present invention, the processes to ethanol-precipitate, filtrate, centrifuge, and thereafter dry, dissolve by the ion-exchange resin, filtrate, and freeze-dry can be omitted, so that the mucopolysaccharide such as the chondroitin sulfuric acid can be produced in a short time and at a low cost.

It should be noted that if the method to dissolve cartilage of fish is different, the composition of the mucopolysaccharide is also different. A food material formed by the mucopolysaccharide produced according to the present invention does not turn brown when blended with vitamin C.

### Best Mode for Carrying out the Invention

The following description relates to preferred embodiments of the present invention.

First, a nose cartilage is obtained by eliminating and separating the epidermis, bone, meat, and so forth from the head portion of a salmon.

Next, the nose cartilage is dissolved to obtain an aqueous solution. Here, the aqueous solution can be obtained by treating the nose cartilage with alkali or enzyme. It is also possible to dissolve the nose cartilage by pressurizing it at about 120°C (1.2 atmosphere) for about one hour.

Subsequently, a proteolytic enzyme (protease) is blended into the aqueous solution obtained.

After that, the insoluble matter being eliminated, the aqueous solution is added with the filter aid, and filtered by the filter press. Here, if deodorization is necessary, an activated carbon is added to perform filtrating by the filter press.

Thereafter, the aqueous solution is spray-dried by the spray-dry technique. It may be concentrated before being dried. Hence, the chondroitin sulfuric acid can be produced in a short time and at a low cost.

The chondroitin sulfuric acid thus produced is the so-called mucopolysaccharide, and according to an analytical result, it has chondroitin sulfuric acid of 20 to 60 wt on average, collagen of 10 to 40 wt % on average, and amino acid of 20 to 40 wt % found therein. Further, a small amount of hyaluronic acid and glucosamine are also contained therein.

Further, according to another embodiment of the present invention, cartilage of fish is dissolved using at least one of alkali or enzyme so as to obtain an aqueous solution, which is then pH-adjusted, filtrated, defatted, deodorized, decolorized, and dried.

Specifically, first, cartilage of salmon is cleaned in a warm water of 40°C to 50°C for one to two hours (defatting and deodorizing). Here, hydrochloric acid may be used for the cleaning (defatting and deodorizing). At that time, the cartilage may have bone, epidermis, and meat adhered thereto. A pressure-treatment and a high-temperature treatment may cause a change in color of a product.

Subsequently, the cartilage is added with water in an amount identical thereto, and the obtained aqueous solution is added with a proteolytic enzyme (aroase) having a ratio of 0.2 % with respect to the concentration of the solid content, which is stirred at 50°C to 60°C for four hours, so as to obtain an aqueous solution.

Thereafter, the aqueous solution is heated at 95° C for five minutes so as to deactivate the enzyme.

Further, the material is added with an activated carbon the weight of which amounting to 2.5 % with respect to that of the material, and stirred at 40°C to 50°C for one to two hours so as to be defatted, deodorized, and decolorized.

Subsequently, the aqueous solution is adjusted so as to have a pH of five to six using acetic acid.

Then the aqueous solution is added with a filter aid, and filtrated by the filter press (defatting). The obtained aqueous solution may be concentrated. After letting the aqueous solution stand, any oil content that floats is separated (defatting).

Subsequently, the aqueous solution is dried by the spray-dry technique.

The mucopolysaccharide thus obtained contains 40% of chondroitin sulfuric acid, 20 % of collagen, and other components such as amino acid, hyaluronic acid, and glucosamine.

Alternatively, cartilage of salmon is cleaned in a warm water of 40°C to 50°C for one to two hours (defatting and deodorizing). Here, hydrochloric acid may be used for the cleaning (defatting and deodorizing). At that time, the cartilage may have bone, epidermis, and meat adhered thereto. A pressure-treatment and a high-temperature treatment may cause a change in color of a product.

Subsequently, water is added to the cartilage in an amount half thereof, so that an aqueous solution is obtained. The aqueous solution is added with 0.1 wt % of a proteolytic enzyme (pancreatin) and stirred at 40°C to 50°C for one hour.

Thereafter, the aqueous solution is heated at 90°C or higher for ten minutes so as to deactivate the enzyme.

Subsequently, the aqueous solution is adjusted so as to have a pH of five to six using acetic acid.

The aqueous solution is then added with a filter aid, and filtrated by the filter press after being deodorized. The obtained aqueous solution may be concentrated. After letting the aqueous solution stand, any oil content that floats is separated (defatting).

Subsequently, alcohol of 50 wt % concentration is added to the aqueous solution while being stirred, and the precipitate is collected.

The collected precipitate is then dried under reduced pressure.

The mucopolysaccharide thus obtained contains 60 wt % of chondroitin sulfuric acid, 10 wt % of collagen, and other components such as amino acid, hyaluronic acid, and glucosamine.

Alternatively, cartilage of salmon is cleaned in a warm water of 40°C to 50°C for one to two hours (defatting and deodorizing). Here, hydrochloric acid may be used for the cleaning (defatting and deodorizing). At that time, the cartilage may have bone, epidermis, and meat adhered thereto. A pressure-treatment and a high-temperature treatment may cause a change in color of a product.

Subsequently, alkali (caustic soda) of 25 wt % concentration is added to the cartilage in an amount of 2 wt % with respect to the cartilage, so that an aqueous solution is obtained. The aqueous solution is stirred at 50°C to 60°C for four hours.

Subsequently, the aqueous solution is adjusted so as to have a pH of six to seven using acetic acid.

Further, the aqueous solution is added with an activated carbon amounting to 0.3 wt % with respect thereto, and stirred for 15 minutes at 80°C, whereby enzyme deactivation, defatting, deodorizing, and decolorizing are performed.

The aqueous solution is then added with a filter aid, and filtrated by the filter press (defatting). The obtained aqueous solution may be concentrated. After letting the aqueous solution stand, any oil content that floats is separated (defatting).

Subsequently, the aqueous solution is dried by the spray-dry technique.

The mucopolysaccharide thus obtained contains 20 % of chondroitin sulfuric acid, 30 % of collagen, and other components such as amino acid, hyaluronic acid, and glucosamine.

### Examples

### (Example 1)

First, a nose cartilage is obtained by eliminating and separating the epidermis, bone, meat, and so forth from the head portion of a salmon. Subsequently, the material (nose cartilage) is added with water amounting to one and a half thereof, and pressured and heated at 120°C for one hour so as to be dissolved, whereby an aqueous solution is obtained. After that, the insoluble matter being eliminated, the aqueous solution is added with a proteolytic enzyme (protease) of 0.2 wt % with respect to the concentration of the solid content, and stirred at 60°C for one hour. The aqueous solution is then heated at 95°C for one hour so as to deactivate the enzyme. Further, the aqueous solution is added with the activated carbon with the weight amounting to 0.3 wt % with respect to that of the aqueous solution, and stirred at 80°C for 15 minutes (defatting, deodorizing, and decolorizing). Subsequently, the aqueous solution is added with a filter aid and filtrated by the filter press, and the filtrated aqueous solution is spray-dried, so that the mucopolysaccharide is obtained. According to the analytical value of the obtained mucopolysaccharide, drying loss of 7 wt %, ash content of 5 wt %, salinity of 0.2 wt %, fat of 0.3 wt %, total nitrogen of 11 wt %, protein of 62 wt %, chondroitin sulfuric acid of 30 wt %, and collagen of 40 wt % are contained.

### (Example 2)

First, cartilage of salmon is cleaned in a warm water of 50°C for two hours. The cartilage is added with water in an amount identical thereto, and a proteolytic enzyme (aroase) amounting to 0.2 wt % of the material, and stirred at 50°C for four hours, so as to obtain an aqueous solution. Thereafter, the aqueous solution is heated at 95°C for five minutes so as to deactivate the enzyme. Further, the material is added with the activated carbon with the weight amounting to 2.5 wt % with respect thereto, and stirred at 50°C for two hours so as to be defatted, deodorized, and decolorized. Subsequently, the aqueous solution is adjusted so as to have a pH of six, added with a filter aid, and filtrated by the filter press (defatting). Subsequently, the aqueous solution is dried by the spray-dry technique so that the mucopolysaccharide is obtained. The mucopolysaccharide thus obtained contains 40 wt % of chondroitin sulfuric acid, 20 wt % of collagen, and other components such as amino acid, hyaluronic acid, and glucosamine. The yield is 6.5 %.

### (Example 3)

First, cartilage of salmon is cleaned in a warm water of 45°C for two hours (defatting and deodorizing). The cartilage is added with water in an amount half thereof, and further added with 0.1 wt % of a proteolytic enzyme (pancreatin), being stirred at 50°C for two hours to obtain an aqueous solution. Thereafter, the aqueous solution is heated at 90°C or higher for ten minutes so as to deactivate the enzyme. The aqueous solution is then adjusted so as to have a pH of six using acetic acid, and being added with a filter aid, filtrated by the filter press after being deodorized. Further, the aqueous solution is added with alcohol of 50 wt % concentration while being stirred, and the precipitate is collected. The collected precipitate is then dried under reduced pressure. The mucopolysaccharide thus obtained contains 60 wt % of chondroitin sulfuric acid, 10 wt % of collagen, and other components such as amino acid, hyaluronic acid, and glucosamine. The yield is 3 %.

### (Example 4)

First, cartilage of salmon is cleaned in a warm water of 45°C for two hours (defatting and deodorizing). Subsequently, the cartilage is added with alkali (caustic soda) of 25 wt % concentration in an amount of 2 wt % with respect to the amount of the cartilage, and stirred at 55°C for four hours so that an aqueous solution is obtained. Subsequently, the aqueous solution is adjusted so as to have a pH of seven using acetic acid. Further, the aqueous solution is added with an activated carbon amounting to 0.3 wt % with respect thereto, and stirred for 15 minutes at 80°C, whereby enzyme deactivation, defatting, deodorizing, and decolorizing are performed. The aqueous solution is then added with a filter aid and filtrated by the filter press (defatting), and dried by the spray-dry technique. The mucopolysaccharide thus obtained contains 20 wt % of chondroitin sulfuric acid, 30 wt % of collagen, and other components such as amino acid, hyaluronic acid, and glucosamine. The yield is 5 %.

### (Example 5)

A nose cartilage is obtained by eliminating and separating the epidermis, bone, meat, and so forth from the head portion of a salmon. The material (nose cartilage) is added with water amounting to one and a half thereof, pressured and heated at 120°C for one hour so as to be dissolved, whereby an aqueous solution is obtained. After that, the insoluble matter being eliminated, the aqueous solution is added with alkali (caustic soda) of 25 wt % concentration in an amount of 2 wt % with respect to the amount of the cartilage, and stirred at 55°C for four hours. The aqueous solution is then adjusted so as to have a pH of seven by acetic acid, added with the activated carbon with the weight amounting to 0.3 wt % with respect to the aqueous solution, and stirred at 80°C for 15 minutes, whereby enzyme deactivation, defatting, deodorizing, and decolorizing are performed. Subsequently, the aqueous solution is added with a filter aid, filtrated (defatted) by the filter press, and dried by the spray-dry technique. The obtained mucopolysaccharide contains 20 wt % of chondroitin sulfuric acid, 30 wt % of collagen, and other components such as amino acid, hyaluronic acid, and glucosamine. The yield is 6 %.

Next, a health food (tablet) composed mainly of the mucopolysaccharide (containing 60 wt % of chondroitin) produced according to the present invention is prepared, and actually tried by human beings to examine the result. The mucopolysaccharide contains 60 wt % of chondroitin sulfuric acid, 10 wt % of collagen, 20 wt % of amino acid, and 2.0 wt % of hyaluronic acid. Twelve tablets (3.6 g) each weighing 300 mg are ingested, each tablet containing: 8.0 wt % of Vitamin C, 7.0 wt % of beer yeast, 45.5 wt % of lactose, 10.0 wt % of indigestible dextrin, and 3.0 wt % of sucrose fatty ester, with respect to the mucopolysaccharide. The ingestion period is 30 days. The results are shown in Table 1.

**Table 1**

| Name (in initial) | Age | Gender | Result |
|---|---|---|---|
| MS | 20s | female | In about one week of ingestion, the skin feels moisturized |
| YU | 20s | female | In about 10 days, a friend told that my skin was getting smoother recently. |
| RA | 20s | female | Constipation is got over. |
| AO | 30s | female | In the morning when getting up, the skin felt supple. After makeup, the skin has a smooth texture. |
| TW | 30s | female | Getting a little slimmer. |
| YO | 30s | female | Pimples do not break out at the period of menstruation. |
| MK | 30s | male | Diuretic. |
| YI | 30s | male | The pain of the knee joint caused at a marathon is getting better. |
| YK | 40s | female | Relieving from arthritis pain. |
| KN | 40s | female | Felt as getting energetic. |
| MI | 40s | female | Skin feels supple. |
| KT | 40s | male | Bleary eyes are cured. |
| MK | 40s | male | Breaking free from fatigue. |
| HY | 50s | female | Pain of the joint is gone. Hangnail is cured. |
| NT | 50s | female | Skin feels supple. |
| MK | 50s | female | Pain of the joint is gone. |
| HM | 60s | female | Pain of the joint is gone. |
| TS | 60s | female | Skin feels healthier. |
| TK | 60s | male | Pain of the joint is gone. |

### Industrial Availability

According to the present invention, a mucopolysaccharide can be produced at a low cost and in a short time. Further, a quality cosmetic material or food material composed of chondroitin sulfuric acid, collagen, amino acid, hyaluronic acid, and glucosamine can be produced from one material.

## Claims

1. A production method of a mucopolysaccharide, said production method comprising the steps of:
treating cartilage of fish with enzyme or alkali to obtain an aqueous solution; and
thereafter defatting, deodorizing, decolorizing, filtrating, and drying the aqueous solution.

2. A mucopolysaccharide produced by the production method comprising the steps of:
treating cartilage of fish with enzyme or alkali to obtain an aqueous solution; and
thereafter defatting, deodorizing, decolorizing, filtrating, and drying the aqueous solution.

3. The mucopolysaccharide according to claim 2, which is a cosmetic material or a food material.

4. A production method of a mucopolysaccharide, said production method comprising the steps of:
pressure-treating cartilage of fish to obtain an aqueous solution;
treating the aqueous solution with enzyme or alkali; and
thereafter defatting, deodorizing, decolorizing, filtrating, and drying the aqueous solution.

5. A mucopolysaccharide produced by the production method comprising the steps of:
pressure-treating cartilage of fish to obtain an aqueous solution;
treating the aqueous solution with enzyme or alkali; and
thereafter defatting, deodorizing, decolorizing, filtrating, and drying the aqueous solution.

6. The mucopolysaccharide according to claim 5, which is a cosmetic material or a food material.
